# EUROPÄISCHE PATENTANMELDUNG

(11) **EP 0 997 119 A2**
(43) Veröffentlichungstag der Anmeldung: **03.05.2000**
(21) Anmeldenummer: 99121708.4
(22) Anmeldetag: 02.11.1999
(51) Int. Cl.: A61F 2/78

(54) **Vorrichtung zum Anlegen einer Prothese**

(30) Priorität: 30.10.1998 DE 29819424 U
(71) Anmelder: Sommer, Claus, 86701 Rohrenfels (DE)
(72) Erfinder: Sommer, Claus, 86701 Rohrenfels (DE)
(74) Vertreter: Witzany, Manfred

(57) **Zusammenfassung**

Eine Vorrichtung (1) dient zum Anlegen einer Prothese (2), welche einen Schaft (3) zur Aufnahme eines Stumpfes (5) aufweist. Im Schaft (3) ist eine Einstecköffnung (4) zum Einführen des mit einem Schlauch (7) überzogenen Stumpfes (5) vorgesehen. Am gegenüberliegenden Ende des Schaftes (3) ist eine Öffnung (6) zum Herausziehen des Schlauches (7) aus dem Schaft (3) vorgesehen. Die Vorrichtung (1) vereinfacht das Herausziehen des Schlauches (7) aus dem Schaft (3). Hierzu weist die Vorrichtung (1) ein Gestell (11) auf, an dem eine den Schlauch (7) umlenkende Welle (13) drehbar gehalten ist, die mit einem Drehantrieb (14) in Wirkverbindung steht. Am Gestell (11) ist außerdem ein Widerlager (25) zur seitlichen Abstützung der Prothese (2) vorgesehen.

## Beschreibung

Die Erfindung betrifft eine Vorrichtung zum Anlegen einer Prothese gemäß dem Oberbegriff des Patentanspruchs 1.

Aus der Praxis sind Prothesen für Arm- oder Beinamputierte bekannt, die einen Schaft mit einer Einstecköffnung zum Einführen eines Stumpfes der amputierten Person aufweisen. Um einen guten Halt der Prothese am Stumpf sicherzustellen, ist diese üblicherweise innenseitig mit einer gut haftenden Schicht, vorzugsweise Gummi oder Leder, belegt. Um trotz dieser haftenden Schicht zu ermöglichen, daß der Stumpf durch die Einstecköffnung in den Schaft vollständig eingeführt werden kann, wird der Stumpf vorher mit einem Schlauch überzogen. Dieser Schlauch besteht vorzugsweise aus Kunststoff oder Glasfasergewebe und ist in Längsfalten gelegt. Dieser Schlauch vermindert erheblich die Reibung des Stumpfes gegenüber dem Schaft, so daß der Stumpf bequem eingeführt werden kann. Um nach dem Einführen des Stumpfes in den Schaft eine ausreichende Haltewirkung der Prothese sicherzustellen, muß der Schlauch anschließend entfernt werden. Hierzu ist es bekannt, am Schaft eine Öffnung vorzusehen, die der Einstecköffnung gegenüberliegt. Eine mit dem Schlauch verbundene Schnur wird vor dem Einstecken des Stumpfes in den Schaft der Prothese durch diese Öffnung gefädelt, so daß der Schlauch mit Hilfe dieser Schnur durch die Öffnung des Schaftes herausgezogen werden kann. Da der Schlauch zwischen dem Stumpf und der haftenden Schicht des Schaftes klemmend gehalten ist, muß entsprechend stark an der Schnur gezogen werden, um den Schlauch durch die Öffnung zu entfernen. Dies ist eine mühselige Arbeit, zu der grundsätzlich eine Hilfsperson erforderlich ist. Steht aber keine Hilfsperson zur Verfügung, so muß die amputierte Person selbst versuchen, den Schlauch mit Hilfe der Schnur durch die Öffnung des Schaftes herauszuziehen. Dies ist insbesondere beim Anlegen einer Beinprothese sehr schwierig. In diesem Fall wird die Schnur vollständig umlenkend aus der Austrittsöffnung gezogen, was zu einer erhöhten Reibung der Schnur und des Schlauches an der Austrittsöffnung führt.

Aus der US-A-3,922,727 ist eine Vorrichtung zum Anlegen einer Prothese bekannt, die im wesentlichen von einer Platte und einem darauf gehaltenen Motor gebildet ist. Der zwischen Stumpf und Schaft vorgesehene Schlauch wird durch eine Öffnung am unteren Ende des Stumpfes herausgezogen und auf der Antriebswelle des Motors aufgewickelt. Dabei wird der Schlauch im Bereich der Öffnung S-förmig umgelenkt, was zu einer erhöhten Reibung und einer entsprechenden seitlichen Zugkraft auf die Prothese führt.

Der Erfindung liegt daher die Aufgabe zugrunde, eine Vorrichtung zu schaffen, die einer amputierten Person ohne Zuhilfenahme einer Hilfsperson das Anlegen einer Prothese erleichtert.

Diese Aufgabe wird erfindungsgemäß mit den Merkmalen der Patentansprüche 1 oder 2 gelöst.

Die erfindungsgemäße Vorrichtung gemäß Anspruch 1 weist eine Welle zum Aufwickeln der Schnur und des Schlauches auf, die mit einem Drehantrieb in Wirkverbindung steht. Damit wird der Schlauch durch die Kraftwirkung des Drehantriebs aus der Öffnung des Schaftes herausgezogen, was für die amputierte Person eine erhebliche Erleichterung darstellt. Die Welle kann dabei derart angeordnet werden, daß der Schlauch nicht mehr in etwa in der Ebene der Öffnung des Schaftes gezogen wird, so daß die Reibung des Schlauches an der Öffnung gering bleibt. Der Schlauch kann daher mit entsprechend verringerter Zugkraft durch die Öffnung herausgezogen werden, was die Gefahr, den Schlauch zu zerreißen, entsprechend reduziert. Da der Schlauch im Bereich der Öffnung nicht mehr um 180° geknickt wird, sind auch Verschleißerscheinungen am Schlauch reduziert, was sich vorteilhaft auf dessen Lebensdauer auswirkt. Damit ist die amputierte Person zum Anlegen der Prothese nicht mehr auf eine Hilfsperson angewiesen. Zur Erhöhung der Reibung zwischen dem Schlauch und der Welle kann der Schlauch auch mehrfach über die Welle geschlungen werden. Um das Anlegen der Prothese weiter zu erleichtern, ist der Welle ein Widerlager zugeordnet. An diesem Widerlager kann die Prothese abgestützt werden, so daß die von der Welle über den Schlauch auf die Prothese ausgeübte Zugkraft nicht durch die amputierte Person abgestützt werden muß. Das Widerlager ist vorzugsweise nahe der Auflauflinie des Schlauches auf die Welle angeordnet, so daß die von der Welle ausgeübte Zugkraft möglichst vollständig vom Widerlager aufgenommen wird, ohne ein wesentliches Drehmoment auf die Prothese auszuüben.

Bei der Vorrichtung gemäß Anspruch 2 ist die Welle derart angeordnet, daß der Schlauch in etwa fluchtend zur Richtung der Öffnung herausgezogen wird. Der Schlauch wird in diesem Fall im Bereich der Öffnung in keiner Weise umgelenkt, so daß er besonders schonend behandelt wird. Vorzugsweise schließt die Öffnung mit der Schaftachse einen stumpfen Winkel ein, um die Schlauchreibung auch innerhalb des Schaftes zu reduzieren. Damit kann die Zugkraft, die die Welle auf den Schlauch ausübt, auf ein Minimum reduziert werden, so daß die Person den Schlauch auch mit eigener Kraft, ggf. über ein untersetzendes Getriebe, durch die Öffnung hindurchziehen kann. Durch die verminderte, auf den Schlauch ausgeübte Zugkraft werden auch die auf die Prothese ausgeübten Kräfte entsprechend reduziert.

Eine besonders günstige Kraftübertragung ergibt sich gemäß Anspruch 3, wenn der Schlauch auf der Welle aufgewickelt wird.

Zur Erzielung einer guten Kraftübertragung zwischen der Welle und dem Schlauch ist es gemäß Anspruch 4 günstig, an der Welle Haltemittel vorzusehen. Diese Haltemittel erfassen eine mit dem Schlauch verbundene Schnur, mit deren Hilfe der Schlauch einfach aus dem Schaft herausgezogen wird.

Vorzugsweise werden die Haltemittel gemäß Anspruch 5 von einer hinterschnittenen Nut gebildet, die in der Welle vorgesehen ist. Damit kann die Schnur einfach in die Nut eingelegt werden, die sich beispielsweise durch einen Knoten an ihrem Ende in der Nut verankert. Durch diese Maßnahme stehen die Haltemittel in keiner Weise über die Welle über, so daß der Schlauch bündig an der Wellenoberfläche anliegend aufgewickelt werden kann, ohne den Schlauch zu beschädigen.

Um die auf den Schlauch bzw. auf die Prothese ausgeübte Zugkraft in ihrer Richtung an die individuellen Bedürfnisse der jeweiligen Prothese anpassen zu können, ist es gemäß Anspruch 6 vorteilhaft, die Welle bzw. das Widerlager verstellbar zu halten. Durch eine Höhenanpassung der Welle läßt sich eine besonders günstige Ziehrichtung des Schlauches einstellen, so daß der Schlauch besonders schonened aus dem Schaft der Prothese herausgezogen wird. Durch Anpassung der Winkellage der Wellenachse läßt sich erreichen, daß der Schlauch gleichmäßig auf die Welle aufgewickelt wird.

Gemäß Anspruch 7 ist es günstig, wenn die Vorrichtung eine Platte aufweist, auf der die Prothese abstützbar ist. Damit wird die von der Welle auf die Prothese ausgeübte Zugkraft ausreichend abgestützt, so daß ein Verankern der Vorrichtung im Boden entfallen kann. Vorzugsweise ist die Platte so groß ausgebildet, daß sich die Person mit ihrem Gesamtgewicht, also mit beiden Beinen, darauf abstützen kann, um zu der von der Welle ausgeübten Zugkraft eine ausreichende Gegenkraft zu erzeugen.

Um stets eine definierte Kraftrichtung der von der Welle ausgeübten Zugkraft zu erzielen, ist es vorteilhaft, wenn die Prothese auf der Vorrichtung stets in gleicher Weise positioniert wird. Dies wird gemäß Anspruch 8 durch eine auf der Platte vorgesehene Markierung erleichtert, der ggf. an ihrer der Welle zugewandten Seite ein Anschlag zugeordnet sein kann.

Um den Drehantrieb einfach steuern zu können, ist es gemäß Anspruch 9 vorteilhaft, eine Bedienungskonsole vorzusehen. Diese Bedienungskonsole ist vorzugsweise in Hüfthöhe vorgesehen, damit sie von der amputierten Person leicht bedient werden kann.

Schließlich ist es gemäß Anspruch 10 günstig, wenn der Drehantrieb richtungsumkehrbar oder auskuppelbar ist. Durch diese Maßnahme kann der auf der Welle aufgewickelte Schlauch leicht wieder abgewickelt werden, indem der Drehantrieb in umgekehrter Richtung angetrieben oder ausgekuppelt wird.

Die Erfindung wird beispielhaft anhand der Zeichnung beschrieben, ohne den Schutzumfang zu beschränken.

Es zeigt:
- Figur 1: eine räumliche Darstellung einer Vorrichtung zum Anlegen einer Prothese,
- Figur 2: eine Ansicht eines Ausschnitts der Welle und
- Figur 3: eine zugeordnete Schnittdarstellung zu Figur 2.

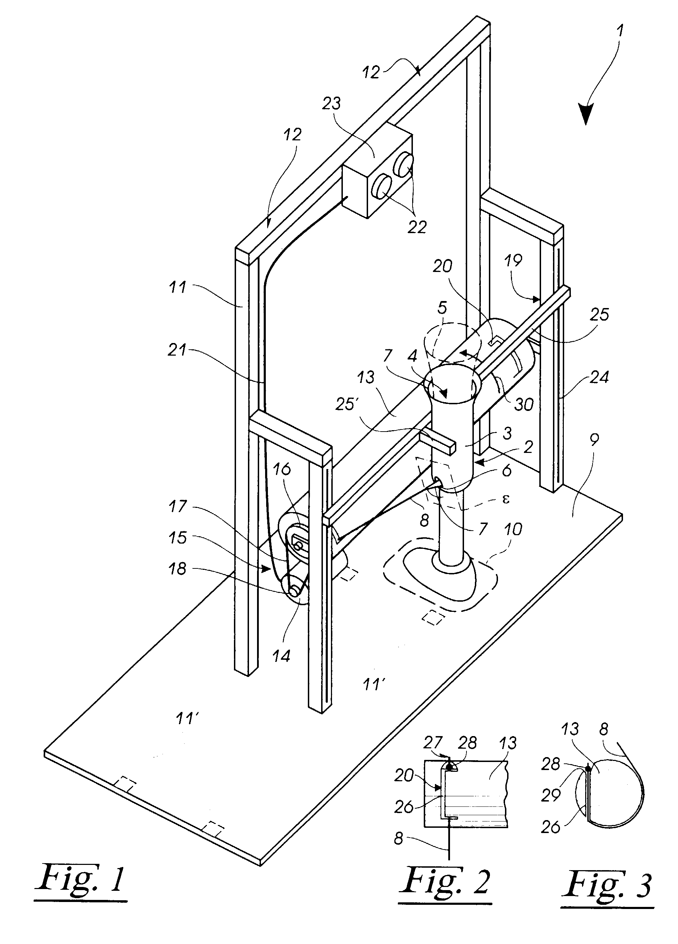

Eine Vorrichtung 1 gemäß Figur 1 dient zum Anlegen einer Prothese 2. Die Prothese 2 wird von einem Schaft 3 gebildet, der eine oberseitige Einstecköffnung 4 für einen strichliert angedeuteten Stumpf 5 aufweist. Der Schaft 3 weist eine der Einstecköffnung 4 gegenüberliegende Öffnung 6 auf, durch die ein Schlauch 7 aus dem Schaft 3 herausgezogen werden kann. Der Schlauch 7 dient als Gleithilfe zum Einstecken des Stumpfes 5 in den Schaft 3, der innenseitig mit Gummi belegt ist. Um den Schlauch 7 durch die Öffnung 6 aus dem Schaft 3 herausziehen zu können, ist an dessen Ende eine Schnur 8 befestigt.

Die Vorrichtung 1 weist eine Platte 9 auf, auf die sich eine Person mit einem nicht dargestellten Fuß und der Prothese 2 stellen kann. Um eine definierte Ausrichtung der Prothese 2 zur Vorrichtung 1 zu erzielen, damit diese paßgenau am Stumpf 5 sitzt, ist in der Platte 9 eine Markierung 10 vorgesehen, der ggf. ein nicht dargestellter Anschlag zugeordnet sein kann. Auf der Platte 9 ist ein Gestell 11 gehalten, das im oberen Bereich als Haltegriff 12 für die Person ausgebildet ist. Das Gestell 11 ist lösbar mit der Platte 9 verbunden und neben der dargestellten Lage auch in einer Position 11' an der Platte 9 festlegbar. Hierdurch läßt sich die Vorrichtung 1 an die Bedürfnisse der Person anpassen, um eine Prothese für das linke bzw. rechte Bein anlegen zu können.

Am Gestell 11 ist eine Welle 13 drehbar abgestützt, die von einem Drehantrieb 14 in Form eines Elektromotors über ein Getriebe 15 angetrieben ist. Das Getriebe 15 wird von einem drehfest mit der Welle 13 verbundenen Zahnrad 16 gebildet, in das ein Zahnriemen 17 eingreift, der von einem Ritzel 18 des Drehantriebs 14 angetrieben ist. Die Welle 13 ist zusammen mit dem Drehantrieb 14 entlang einer am Gestell 11 vorgesehenen Führung 19 höhen- und winkelverstellbar gehalten. Die Welle 13 ist mit Haltemitteln 20 zum Erfassen der Schnur 8 ausgestattet.

Der Drehantrieb 14 ist über eine elektrische Leitung 21 mit einer Bedienkonsole 22 verbunden, die im Bereich der Haltegriffe 12 am Gestell 11 festgelegt ist. Die Bedienkonsole 22 weist zwei Drucktasten 23 auf, mit deren Hilfe der Drehantrieb 14 drehrichtungsumkehrbar gesteuert werden kann. Durch die Lage der Bedienkonsole 22 im Bereich der Haltegriffe 12 kann die Person den Drehantrieb steuern, ohne die Haltegriffe 12 loslassen zu müssen.

Am Gestell 11 ist eine weitere Führung 24 vorgesehen, entlang der ein Widerlager 25 in Form eines Querträgers höhen- und winkelverstellbar gehalten ist. An diesem Widerlager 25 kann die Prothese 2 abgestützt werden, um der von der Welle 13 ausgeübten Zugkraft zu widerstehen. Am Widerlager 25 ist in etwa mittig eine Nase 25' gehalten, mit der längs des Trägers 25 wirkende Zugkräfte abgestützt werden. Alternativ könnte das Widerlager in diesem Bereich auch an die Form der Prothese 2 angepaßt sein.

Figur 2 zeigt einen Endbereich der Welle 13 mit den Haltemitteln 20 zur Festlegung der Schnur 8. Die Haltemittel 20 sind von einer hinterschnittenen Nut 26 gebildet, in die die Schnur 8 eingelegt werden kann. Im Bereich des Schnurendes 27 ist ein Knoten 28 vorgesehen, der nicht in die Nut 26 eindringen kann. Auf diese Weise ergibt sich eine kraftschlüssige Verbindung der Welle 13 mit der Schnur 8. Am Gegenende der Welle 13 sind die Haltemittel 20 spiegelbildlich vorgesehen, um das Anlegen von linken und rechten Prothesen 2 zu erleichtern.

Figur 3 zeigt eine Schnittdarstellung der Welle 13 durch die Nut 26. Aus dieser Darstellung ist insbesondere zu entnehmen, daß die Schnur 8 die Welle 13 durchsetzt, was zu einer besonders günstigen, kraftschlüssigen Verbindung zwischen beiden führt. Der Knoten 28 der Schnur 8 ist in einer Vertiefung 29 der Welle 13 gehalten, so daß dieser nicht über den Außenumfang der Welle 13 übersteht. Der Schlauch 7 kann demnach schonend, auf der Oberfläche der Welle 13 bündig aufliegend, aufgewickelt werden.

Im folgenden wird das Verfahren zum Anlegen der Prothese 2 anhand der Figuren erläutert. Je nachdem, ob eine linke oder rechte Prothese 2 anzuziehen ist, wird das Gestell 11 in der Position 11' oder in der dargestellten Lage an der Platte 9 festgelegt. Die amputierte Person zieht zunächst den Schlauch 7 über ihren Stumpf 5, um die Reibung zwischen dem Stumpf 5 und dem Schaft 3 der Prothese 2 zu verringern. Dies ist wichtig, da der Schaft 3 innenseitig mit Gummi belegt ist, um eine ausreichende Haftung des Schaftes 3 am Stumpf 5 zu gewährleisten. Dieses gewünschte Haftvermögen ist jedoch beim Anlegen der Prothese 2 so hinderlich, daß der Schlauch 7 zur Verminderung der Reibung unbedingt erforderlich ist. Der Schlauch 7 besteht vorzugsweise aus Glasfasermaterial, das in Längsfalten gelegt ist. Damit weist der Schlauch 7 eine ausreichende Festigkeit auf und vermindert die Reibung im erforderlichen Maß.

Die am unteren Ende des Schlauches 7 befestigte Schnur 8 wird durch die Einstecköffnung 4 in den Schaft 3 eingeführt und am Gegenende des Schaftes 3 durch die Öffnung 6 ausgefädelt, so daß die Schnur 8 den Schaft 3 durchsetzt. Anschließend steckt die amputierte Person den mit dem Schlauch 7 überzogenen Stumpf 5 in den Schaft 3 der Prothese 2 ein, was durch die Reibungsverminderung aufgrund des Schlauches 7 erheblich erleichtert wird. Um einen sicheren Halt der Prothese 2 am Stumpf 5 zu gewährleisten, muß jedoch der Schlauch 7 anschließend wieder aus dem Schaft 3 herausgezogen werden, so daß die innenseitige Gummibeschichtung des Schaftes 3 ausreichenden Halt am Stumpf 5 hat. Hierzu zieht die Person zunächst die Schnur 8 durch die Öffnung 6 soweit heraus, wie dies ohne größeren Kraftaufwand möglich ist.

Die Schnur 8 wird dann entsprechend der Darstellung in den Figuren 2 und 3 in die hinterschnittene Nut 26 der Welle 13 eingelegt, um eine kraftschlüssige Verbindung der Welle 13 mit der Schnur 8 zu gewährleisten. Die amputierte Person stellt sich dann derart auf die Platte 9, daß die Prothese 2 auf die Markierung 10 ausgerichtet ist und das andere Bein auf dem über das Gestell 11 hinausragenden Bereich der Platte 9 abgestellt wird. Dabei liegt die Prothese 2 mit ihrem Schaft 3 am Widerlager 25 an. Durch Betätigen der Drucktaste 23 wird nun der Drehantrieb 14 und somit die Welle 13 in Richtung des Pfeiles 30 in Drehung versetzt. Dadurch wird an der Schnur 8 und dem damit verbundenen Schlauch 7 gezogen, wobei die Ziehrichtung so eingestellt ist, daß der Schlauch 7 etwa in Richtung der Öffnung 6 gezogen wird. Auf diese Weise ergibt sich eine besonders geringe Reibungskraft zwischen dem Schlauch 7 und der Öffnung 6 des Schaftes 3. Außerdem wird auf diese Weise der Schlauch 7 besonders schonend herausgezogen, so daß er öfter verwendbar ist. Vorzugsweise zeigt die Öffnung 6 nach unten, so daß der Schlauch 7 innerhalb des Schaftes 3 stumpfwinkelig umgelenkt wird. Dies vermindert die Reibung des Schlauches 7 an der Öffnung 6 zusätzlich, wobei in diesem Fall die Welle 13 entsprechend tiefer anzuordnen ist.

Nachdem unter fortgesetzter Drehung der Welle 13 der gesamte Schlauch 7 aus dem Schaft 3 herausgezogen wurde, läßt die amputierte Person die Drucktaste 23 los, um den Drehantrieb 14 anzuhalten. Durch Betätigung der anderen Drucktaste 23 läßt sich der Drehantrieb 14 entgegen der Drehrichtung 30 bewegen, so daß der Schlauch 7 mit der Schnur 8 wieder von der Welle 13 abgewickelt werden kann, um ihn später wieder einsetzen zu können. Alternativ wäre es auch vorstellbar, den Drehantrieb 14 auskuppelbar mit der Welle 13 zu verbinden, so daß der Schlauch 7 mit der Schnur 8 einfach von der Welle abgezogen werden kann.

### Bezugszeichenliste

- 1: Vorrichtung
- 2: Prothese
- 3: Schaft
- 4: Einstecköffnung
- 5: Stumpf
- 6: Öffnung
- 7: Schlauch
- 8: Schnur
- 9: Platte
- 10: Markierung
- 11: Gestell
- 11': alternative Gestellposition
- 12: Haltegriff
- 13: Welle
- 14: Drehantrieb
- 15: Getriebe
- 16: Zahnrad
- 17: Zahnriemen
- 18: Ritzel
- 19: Führung
- 20: Haltemittel
- 21: elektrische Leitung
- 22: Bedienkonsole
- 23: Drucktaste
- 24: Führung
- 25: Widerlager
- 25': Nase
- 26: Nut
- 27: Schnurende
- 28: Knoten
- 29: Vertiefung
- 30: Drehrichtung

## Patentansprüche

1. Vorrichtung zum Anlegen einer Prothese (2), insbesondere einer Beinprothese, mit einem zur Aufnahme eines Stumpfes (5) vorgesehenen Schaft (3), der eine Einstecköffnung (4) zum Einführen des mit einem Schlauch (7) überzogenen Stumpfes (5) und eine gegenüberliegende Öffnung (6) zum Herausziehen des Schlauches (7) aus dem Schaft (3) durch Einwirkung einer Zugkraft aufweist, wobei die Vorrichtung (1) eine den Schlauch (7) umlenkende, drehbare Welle (13) aufweist, die mit einem Drehantrieb (14) in Wirkverbindung steht, **dadurch gekennzeichnet,** daß die Welle (13) und der Drehantrieb (14) in einem Gestell (11) abgestützt sind, an dem ein Widerlager (25) zur seitlichen Abstützung der Prothese (2) vorgesehen ist.

2. Vorrichtung nach Anspruch 1, **dadurch gekennzeichnet,** daß die Welle (13) in etwa in Fluchtrichtung zur Längserstreckung der Öffnung (6) vorgesehen ist.

3. Vorrichtung nach Anspruch 1 oder 2, **dadurch gekennzeichnet,** daß die Welle (13) als Aufwickeltrommel für den Schlauch (7) ausgebildet ist.

4. Vorrichtung nach Anspruch 3, **dadurch gekennzeichnet,** daß die Welle (13) Haltemittel (20) zur Erfassung einer mit dem Schlauch (7) in Verlängerung verbundenen Schnur (8) aufweist.

5. Vorrichtung nach Anspruch 4, **dadurch gekennzeichnet,** daß die Haltemittel (20) von einer in der Welle (13) vorgesehenen, hinterschnittenen Nut (26) gebildet sind.

6. Vorrichtung nach mindestens einem der Ansprüche 1 bis 5, **dadurch gekennzeichnet,** daß die Welle (13) und/oder das Widerlager (25) höhen- und/oder winkelverstellbar am Gestell (11) gehalten ist/sind.

7. Vorrichtung nach mindestens einem der Ansprüche 1 bis 6, **dadurch gekennzeichnet,** daß das Gestell (11) auf einer Platte (9) vorgesehen ist, auf der zumindest die Prothese (2) abstützbar ist.

8. Vorrichtung nach Anspruch 7, **dadurch gekennzeichnet,** daß auf der Platte (9) eine Markierung (10) für die Prothese (2) vorgesehen ist.

9. Vorrichtung nach mindestens einem der Ansprüche 1 bis 8, **dadurch gekennzeichnet,** daß die Vorrichtung (1) eine Bedienkonsole (22) aufweist, die mit dem Drehantrieb (14) in steuernder Wirkverbindung steht.

10. Vorrichtung nach mindestens einem der Ansprüche 1 bis 9, **dadurch gekennzeichnet,** daß der Drehantrieb (14) in seiner Drehrichtung (30) umkehrbar ist oder auskuppelbar mit der Welle (13) in Wirkverbindung steht.
